# EUROPEAN PATENT APPLICATION

(11) **EP 4 335 374 A1**
(43) Date of publication of application: **13.03.2024**
(21) Application number: 23196288.7
(22) Date of filing: 08.09.2023
(51) Int. Cl.: A61B 6/00

(54) **X-RAY DIAGNOSTIC APPARATUS AND X-RAY DIAGNOSTIC METHOD**

(30) Priority: 09.09.2022 JP 2022143498
(71) Applicant: Canon Medical Systems Corporation, Tochigi 324-0036 (JP)
(72) Inventor: IKEZAKI, Rie, Otawara-shi, 324-0036 (JP); KURATOMI, Naoko, Otawara-shi, 324-0036 (JP); IWAI, Haruki, Otawara-shi, 324-0036 (JP); KOBAYASHI, Yoshimasa, Otawara-shi, 324-0036 (JP)
(74) Representative: Marks & Clerk LLP

(57) **Abstract**

An X-ray diagnostic apparatus according to an embodiment includes an imaging apparatus including an X-ray tube for generating an X ray and an X-ray detector for detecting an X ray emitted from the X-ray tube and passed through a subject. The X-ray diagnostic apparatus further includes a first obtainment unit (245) for obtaining an examination protocol including an imaging condition corresponding to an examination; a second obtainment unit (246) for obtaining a camera image in which the subject has been captured; and a determination unit (248) for determining whether an examination item corresponding to the examination protocol is executable based on first and second geometric information, the first geometric information including a geometric positional relation between the subject and the imaging apparatus associated with the examination protocol, and the second geometric information including a geometric positional relation between the subject and the imaging apparatus detected based on the camera image.

## Description

### FIELD

Embodiments disclosed in this specification and in the drawings are related to X-ray diagnostic apparatus and X-ray diagnostic methods.

### BACKGROUND

In conventional examinations using X-ray diagnostic apparatuses, a positioning of a subject is prescribed in accordance with an examination purpose or a region to be examined. Moreover, a technique has been disclosed in which a region of the subject who has been positioned as prescribed is recognized by a visible light camera and an image of the region is captured.

However, in this conventional technique, imaging needs to be performed by prescribed positioning even for a subject (for example, a subject who has pain due to a bone fracture or has a physical disability) who is difficult to be positioned as prescribed, a physical burden may thus be placed on the subject, and there is thus a need for further improvement.

### SUMMARY OF THE INVENTION

An X-ray diagnostic apparatus comprising:
an imaging apparatus including
   an X-ray tube configured to generate an X ray; and
   an X-ray detector configured to detect an X ray that has been emitted from the X-ray tube and passed through a subject;
a first obtainment unit (245) configured to obtain an examination protocol including an imaging condition corresponding to contents of an examination for the subject;
a second obtainment unit (246) configured to obtain a camera image in which the subject has been captured; and
a determination unit (248) configured to determine whether at least one examination item corresponding to the examination protocol is executable based on first geometric information and second geometric information, the first geometric information including a geometric positional relation between the subject and the imaging apparatus associated with the examination protocol, and the second geometric information including a geometric positional relation between the subject and the imaging apparatus detected based on the camera image.

The X-ray diagnostic apparatus , further comprising an execution unit (243) configured to execute the at least one executable examination item, wherein when the at least one examination item corresponding to the examination protocol is determined to be executable, the execution unit (243) is configured to execute the executable item.

The X-ray diagnostic apparatus , wherein the determination unit (248) is configured to determine whether the first geometric information and the second geometric information agree with each other, and when the first geometric information and the second geometric information do not agree with each other, determine the at least one examination item corresponding to the examination protocol to be not executable, and the X-ray diagnostic apparatus further comprising
a movement control unit (251) configured to control at least one of the X-ray tube or the X-ray detector such that the imaging apparatus is moved to a position corresponding to the first geometric information.

The X-ray diagnostic apparatus , wherein
the first geometric information includes a body position of the subject, an angle of the X-ray tube, and a position of the X-ray detector, those being associated with the examination protocol, and
the second geometric information includes a body position of the subject, an angle of the X-ray tube, and a position of the X-ray detector, those being detected based on the camera image.

The X-ray diagnostic apparatus , further comprising a calculation unit (250) configured to calculate a movement position for the X-ray tube or the X-ray detector such that the imaging apparatus is moved to the position corresponding to the first geometric information by moving at least one of the X-ray tube or the X-ray detector.

The X-ray diagnostic apparatus , further comprising a display control unit (241) configured to perform control such that movement information about movement of the imaging apparatus to a movement position is displayed on a display unit.

The X-ray diagnostic apparatus , further comprising an optical camera capable of collecting the camera image in a same direction as the X ray illuminates in an X-ray tube retaining unit that retains the X-ray tube.

An X-ray diagnostic method performed by an X-ray diagnostic apparatus that includes an imaging apparatus including an X-ray tube configured to generate an X ray and an X-ray detector configured to detect an X ray that has been emitted from the X-ray tube and passed through a subject, the X-ray diagnostic method comprising:
a first obtainment step of obtaining an examination protocol including an imaging condition corresponding to contents of an examination for the subject;
a second obtainment step of obtaining a captured image in which the subject has been captured; and
a determination step of determining whether at least one examination item corresponding to the examination protocol is executable based on first geometric information and second geometric information, the first geometric information including a geometric positional relation between the subject and the imaging apparatus associated with the examination protocol, and the second geometric information including a geometric positional relation between the subject and the imaging apparatus detected based on the captured image.

The geometric information may include at least one of an angle of the X ray illuminating the subject, positions of the X-ray tube and the X-ray detector relative to the subject, an angle of the X-ray detector relative to the subject, a range that the X ray illuminates the subject, or a distance from the X-ray tube to the subject.

The examination protocol may be information including a group of parameters including an imaged region, an imaging condition, a posture of the subject, a body position of the subject, an angle of the X-ray tube, and a position of the X-ray detector.

The examination protocol may be a data file defining, in accordance with a region of a diagnostic target to be examined or an examination purpose, a series of conditions indicating a setting condition for an X-ray diagnostic apparatus or a medical image processing apparatus, an X-ray condition, and a condition for imaging technique.

### BRIEF DESCRIPTION OF THE DRAWINGS

FIG. 1 is a block diagram illustrating an example of a configuration of an X-ray diagnostic apparatus according to an embodiment;
FIG. 2 is a block diagram illustrating an example of another configuration of the X-ray diagnostic apparatus according to the embodiment;
FIG. 3 is a table illustrating an example of examination protocols according to the embodiment;
FIG. 4 is a diagram illustrating an example of a positional relation between the X-ray diagnostic apparatus and a subject, according to the embodiment;
FIG. 5 is a diagram illustrating the example of the positional relation between the X-ray diagnostic apparatus and the subject, according to the embodiment;
FIG. 6 is a diagram illustrating an example of a positional relation between the X-ray diagnostic apparatus and a subject, according to the embodiment;
FIG. 7 is a diagram illustrating an example of a positional relation between the X-ray diagnostic apparatus and a subject, according to the embodiment;
FIG. 8 is a diagram illustrating the example of the positional relation between the X-ray diagnostic apparatus and the subject, according to the embodiment;
FIG. 9 is a flowchart illustrating an example of a process executed by the X-ray diagnostic apparatus according to the embodiment;
FIG. 10 is a diagram illustrating an example of a positional relation between an X-ray diagnostic apparatus and a subject, according to a modified example;
FIG. 11 is a diagram illustrating an example of a positional relation between the X-ray diagnostic apparatus and a subject, according to the modified example; and
FIG. 12 is a diagram illustrating an example of a positional relation between the X-ray diagnostic apparatus and a subject, according to the modified example.

### DETAILED DESCRIPTION

An X-ray diagnostic apparatus comprising: an imaging apparatus including an X-ray tube configured to generate an X ray; and an X-ray detector configured to detect an X ray that has been emitted from the X-ray tube and passed through a subject; a first obtainment unit (245) configured to obtain an examination protocol including an imaging condition corresponding to contents of an examination for the subject; a second obtainment unit (246) configured to obtain a camera image in which the subject has been captured; and a determination unit (248) configured to determine whether at least one examination item corresponding to the examination protocol is executable based on first geometric information and second geometric information, the first geometric information including a geometric positional relation between the subject and the imaging apparatus associated with the examination protocol, and the second geometric information including a geometric positional relation between the subject and the imaging apparatus detected based on the camera image.

The X-ray diagnostic apparatus according to the embodiment will be described hereinafter while reference is made to the drawings. What is described hereinafter is an example of a case where the X-ray diagnostic apparatus that is an apparatus according to the embodiment is a general X-ray imaging apparatus. With respect to the following embodiment, components assigned with the same reference sign will be assumed to operate similarly to one another and redundant description thereof will thus be omitted as appropriate.

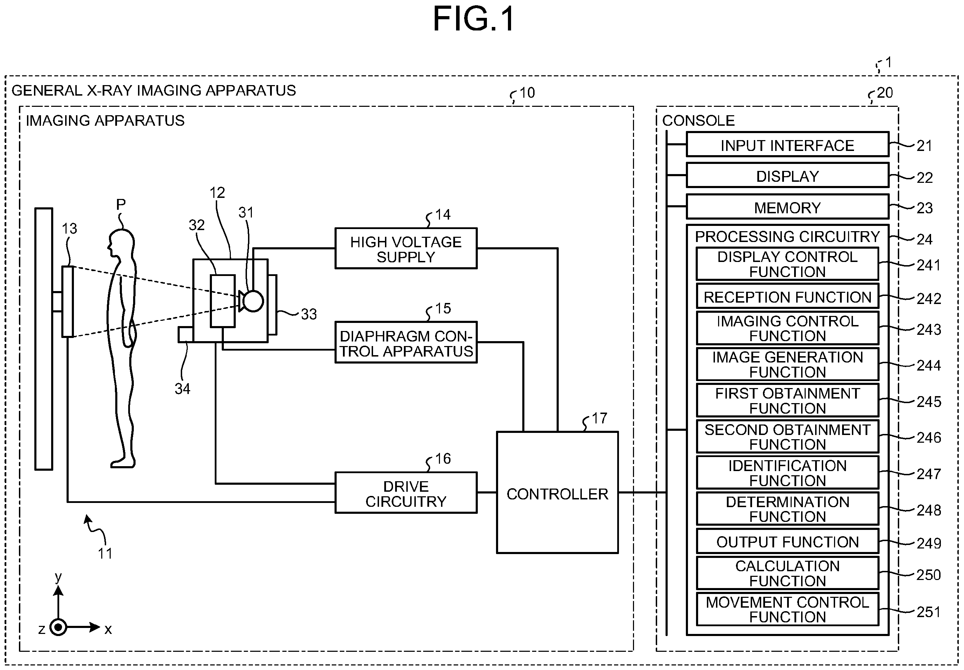

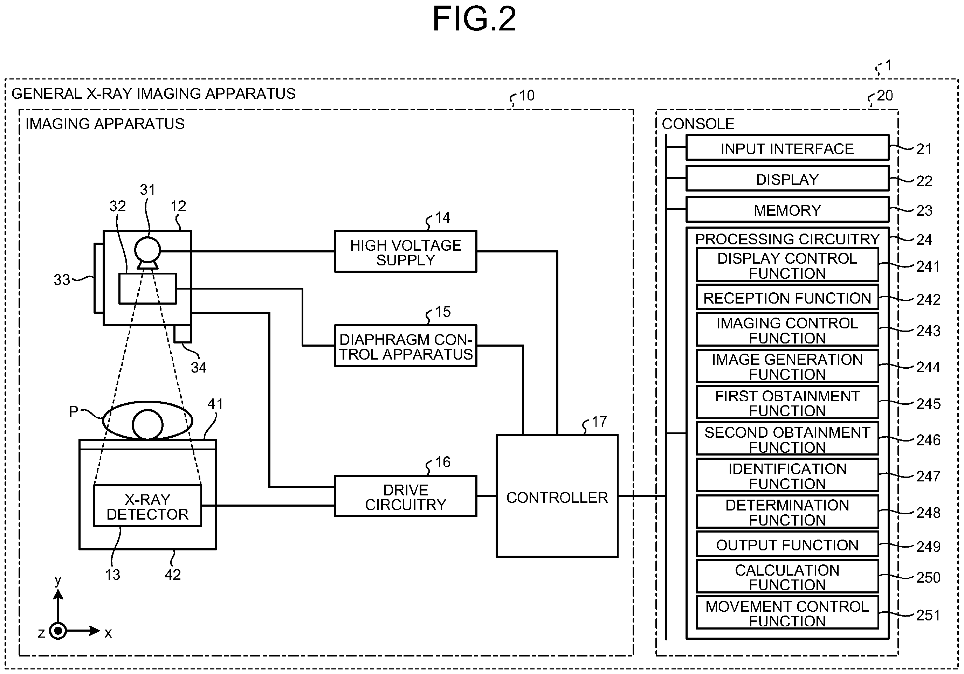

FIG. 1 is a block diagram illustrating an example of a configuration of the X-ray diagnostic apparatus according to the embodiment. FIG. 2 is a block diagram illustrating an example of another configuration of the X-ray diagnostic apparatus. FIG. 1 is an example of a configuration of a general X-ray imaging apparatus 1 for when X-ray imaging of a subject P in a standing posture is performed, and FIG. 2 is an example of a configuration of a general X-ray imaging apparatus 1 for when X-ray imaging of a subject P in a lying posture is performed.

As illustrated in FIG. 1 and FIG. 2, the general X-ray imaging apparatuses 1 each have an imaging apparatus 10 and a console 20. The imaging apparatus 10 is an example of an X-ray imaging unit. The imaging apparatus includes an X-ray tube 31 configured to generate an X ray, and an X-ray detector 13 configured to detect an X ray that has been emitted from the X-ray tube 31 and passed through the subject P. In a case where X-ray imaging of the subject P in the standing posture is performed, as illustrated in FIG. 1, the imaging apparatus 10 has a stand 11, an X-ray tube retaining apparatus 12, the X-ray detector 13 supported movably in relation to the stand 11, a high voltage supply 14, a diaphragm control apparatus 15, drive circuitry 16, and a controller 17.

By contrast, in a case where X-ray imaging of the subject P in the lying posture is performed, as illustrated in FIG. 2, the imaging apparatus 10 has, instead of the stand 11, a couch 42 including a couchtop 41 where the subject P is placed. The couchtop 41 moves up and down by being controlled by the controller 17 via the drive circuitry 16. The couchtop 41 also moves longitudinally (along a Z-axis) in a similar manner.

The X-ray tube retaining apparatus 12 of the imaging apparatus 10 has the X-ray tube 31, a diaphragm 32, an operation panel 33, and a camera 34. The X-ray tube retaining apparatus 12 is an example of an X-ray tube retaining unit.

In the case where X-ray imaging of the subject P in the standing posture is performed, as illustrated in FIG. 1, the subject P who is standing is positioned in front of the stand 11. The X-ray tube retaining apparatus 12 and the X-ray detector 13 are capable of moving in association with each other to maintain the positional relation between the center of the X-ray tube 31 and the X-ray detector 13, by being controlled by the controller 17 via the drive circuitry 16. For example, the X-ray detector 13 supported by the stand 11 moves along the stand 11 in association with movement of the X-ray tube retaining apparatus 12 to maintain the state where the center of the X-ray tube 31 and the approximate center of the X-ray detector 13 are opposed to each other.

In the case where X-ray imaging of the subject P in the lying posture is performed, as illustrated in FIG. 2, the subject P who is lying is placed on the couchtop 41. In this case also, similarly to the case of imaging in the standing posture, the X-ray tube retaining apparatus 12 and the X-ray detector 13 are capable of moving in association with each other to maintain the positional relation between the center of the X-ray tube 31 and the X-ray detector 13, by being controlled by the controller 17 via the drive circuitry 16.

The X-ray detector 13 is a flat panel detector (FPD) having plural X-ray detection elements arranged twodimensionally, detects X-rays that have been transmitted through the subject P and have reached the X-ray detector 13, and outputs, on the basis of the X-rays detected, imaging data on the X-rays. The imaging data are provided to the console 20 via the controller 17. The X-ray detector 13 may include an image intensifier and/or a TV camera, for example.

The X-ray tube 31 is a vacuum tube that emits thermions from a cathode (a filament) to an anode (a target) by application of high voltage from the high voltage supply 14. The X-ray tube 31 is arranged opposite to the X-ray detector 13, with the subject P interposed between the X-ray tube 31 and the X-ray detector 13. The X-ray tube 31 moves in association with movement of the X-ray detector 13 to maintain the state where the center of the X-ray tube 31 and the approximate center of the X-ray detector 13 are opposed to each other, by being controlled by the controller 17 via the drive circuitry 16.

The high voltage supply 14 is electric circuitry including, for example, a transformer and a rectifier, and includes: a high voltage generation apparatus having a function of generating high voltage to be applied to the X-ray tube 31; and an X-ray control apparatus that performs control of output voltage according to X-rays emitted by the X-ray tube 31.

The diaphragm 32 has plural blades formed of a metal that blocks X-rays, the metal being, for example, lead. The diaphragm 32 has a mechanism that adjusts the irradiation field of X-rays generated by the X-ray tube 31. The diaphragm 32 adjusts the range irradiated with X-rays emitted from the X-ray tube 31 by being controlled by the controller 17 via the diaphragm control apparatus 15.

The operation panel 33 is provided on a casing of the X-ray tube retaining apparatus 12 and has: hardware keys, such as buttons, that respectively provide specific instruction signals to a processor when pressed by a user; and a display input apparatus. The display input apparatus has a display serving as a display unit, and a touch sensor provided near the display and serving as an input unit.

The display of the operation panel 33 displays an image representing information related to the general X-ray imaging apparatus 1 and various images including a reference image of the subject P captured by the camera 34. A user is able to input various instructions related to an image displayed on the display, into the general X-ray imaging apparatus 1 via the touch sensor and/or hardware keys of the operation panel 33. The operation panel 33 outputs signals corresponding to the input by the user, to processing circuitry 24 of the console 20.

The camera 34 is provided on the casing of the X-ray tube retaining apparatus 12. The camera 34 is, for example, an optical camera capable of collecting a camera image in the same direction as the X ray illuminates in the X-ray tube retaining apparatus 12 that retains the X-ray tube 31. The camera 34 includes a charge coupled device (CCD) image sensor or a complementary metal oxide semiconductor (CMOS) image sensor. The camera 34 captures an image of a state of the subject P positioned on the stand 11 in the standing posture or the subject P placed on the couchtop 41 in the lying posture and outputs the image captured, to a memory 23.

The camera 34 outputs an image captured, to the processing circuitry 24 of the console 20, via the controller 17. A wide-angle lens or a fish-eye lens may be attached to the camera 34 to enable acquisition of a camera image of a wider range of the subject P. Furthermore, the camera 34 may be provided on a wall surface including the ceiling of the examination room. The camera 34 is a visible light camera. The camera 34 is not necessarily a visible light camera and may be an infrared camera.

The controller 17 has at least a processor and a memory. The controller 17 is controlled by the console 20 according to a program stored in the memory and controls the components of the imaging apparatus 10 integrally.

The console 20 has an input interface 21, a display 22, the memory 23, and the processing circuitry 24. In this embodiment, the console 20 is installed outside the examination room. The console 20 may be not provided independently, and for example, the operation panel 33 of the imaging apparatus 10 may have functions of the input interface 21 and display 22 of the console 20, and the memory and processor of the controller 17 of the imaging apparatus 10 may respectively have functions of the memory 23 and processing circuitry 24.

The input interface 21 of the console 20 includes, for example: a general pointing device, such as a joystick, a trackball, a trackball mouse, a keyboard, a touch panel, or a numeric keypad; and a hand switch for instructing the X-ray exposure timing. The input interface 21 outputs operation signals corresponding to operation performed by a user, to the processing circuitry 24.

The display 22 is a general display output apparatus, such as, for example, a liquid crystal display or an organic light emitting diode (OLED) display, and displays various types of information according to control by the processing circuitry 24.

The memory 23 is an example of a storage unit. The memory 23 is implemented by, for example: a semiconductor memory element, such as a random access memory (RAM) or a flash memory; a hard disk; or an optical disk. The memory 23 stores, for example, imaging data captured by the imaging apparatus 10. The memory 23 also stores examination protocols. Details of the examination protocols will be described later.

The memory 23 stores dedicated programs for implementing a display control function 241, a reception function 242, an imaging control function 243, an image generation function 244, a first obtainment function 245, a second obtainment function 246, an identification function 247, a determination function 248, an output function 249, a calculation function 250, and a movement control function 251, which will be described later.

The processing circuitry 24 controls the overall operation of the general X-ray imaging apparatus 1. The processing circuitry 24 has, for example, the display control function 241, the reception function 242, the imaging control function 243, the image generation function 244, the first obtainment function 245, the second obtainment function 246, the identification function 247, the determination function 248, the output function 249, the calculation function 250, and the movement control function 251.

The display control function 241 is an example of a display control unit. The reception function 242 is an example of a reception unit. The imaging control function 243 is an example of an imaging control unit and an execution unit. The image generation function 244 is an example of an image generation unit. The first obtainment function 245 is an example of a first obtainment unit. The second obtainment function 246 is an example of a second obtainment unit. The identification function 247 is an example of an identification unit. The determination function 248 is an example of a determination unit. The output function 249 is an example of an output unit. The calculation function 250 is an example of a calculation unit. The movement control function 251 is an example of a movement control unit.

Processing functions implemented by the display control function 241, the reception function 242, the imaging control function 243, the image generation function 244, the first obtainment function 245, the second obtainment function 246, the identification function 247, the determination function 248, the output function 249, the calculation function 250, and the movement control function 251, which are components in this embodiment, have been stored in the memory 23, in the form of programs that are able to be executed by a computer.

The processing circuitry 24 is a processor that implements functions corresponding to the programs by reading and executing the programs from the memory 23. In other words, the processing circuitry 24 that has read the programs has the functions illustrated inside the processing circuitry 24 in FIG. 2.

By reference to FIG. 2, the processing functions implemented by the display control function 241, the reception function 242, the imaging control function 243, the image generation function 244, the first obtainment function 245, the second obtainment function 246, the identification function 247, the determination function 248, the output function 249, the calculation function 250, and the movement control function 251 have been described to be implemented by the single piece of processing circuitry 24, but the processing circuitry 24 may include a combination of plural independent processors that implement the functions by executing the programs.

In other words, each of these functions may be configured as a program, and a single piece of processing circuitry may execute each of these programs, or a specific function or specific functions may be implemented by dedicated independent program execution circuitry.

The term, "processor", used in the description above means, for example, a central processing unit (CPU), a graphical processing unit (GPU), or a circuit, such as an application specific integrated circuit (ASIC) or a programmable logic device (for example, a simple programmable logic device (SPLD), a complex programmable logic device (CPLD), or a field programmable gate array (FPGA)).

The processor implements the functions by reading and executing the programs stored in the memory 23. Instead of being stored in the memory 23, the programs may be directly incorporated in a circuit of the processor. In this case, the processor implements the functions by reading and executing the programs incorporated in the circuit.

The display control function 241 performs control to display various types of information, on the display 22.

The reception function 242 receives various instructions from a user. For example, the reception function 242 receives an imaging instruction from the user via the input interface 21.

The imaging control function 243 automatically executes at least one executable examination item corresponding to an examination protocol. The imaging control function 243 is an example of an execution unit. When at least one examination item corresponding to the examination protocol is determined to be executable, the imaging control function 243 executes the executable examination item. For example, the imaging control function 243 controls the X-ray detector 13, the X-ray tube 31, and the diaphragm 32, to perform X-ray imaging of the subject P. Specifically, in a case where the imaging control function 243 has received an imaging instruction from a user, the imaging control function 243 moves, in cooperation with the movement control function 251 described later, the center of the X-ray tube 31 to a position corresponding to an imaged region of the subject P and controls the X-ray tube 31, the X-ray detector 13, and the diaphragm 32 to cause the X-ray tube 31, the X-ray detector 13, and the diaphragm 32 to perform X-ray imaging of the subject P at that position.

Furthermore, in a case where long size imaging is performed, the imaging control function 243 performs X-ray imaging by moving, in cooperation with the movement control function 251, the center of the X-ray tube 31 to positions respectively corresponding to plural imaging areas of an imaging range of a long size image.

The image generation function 244 generates an X-ray image on the basis of X-ray imaging. For example, in a case where long size imaging has been performed, the image generation function 244 generates a long size image on the basis of X-ray imaging performed in each of plural imaging areas. X-ray images and long size images generated by the image generation function 244 are stored in the memory 23.

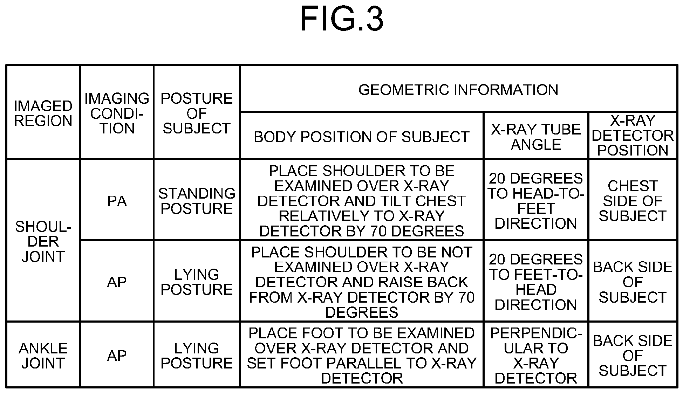

The first obtainment function 245 obtains an examination protocol including an imaging condition corresponding to contents of the examination for the subject P. Specifically, the first obtainment function 245 obtains an examination protocol stored in the memory 23. Description will now be made on this examination protocol by use of FIG. 3. FIG. 3 is a table listing an example of protocols according to the embodiment.

The examination protocols are information prescribing, for example, procedures for controlling the X-ray detector 13, the X-ray tube 31, and the diaphragm 32 and obtaining images by imaging the subject P. The examination protocols are each information including, for example, a group of parameters, such as an imaged region, an imaging condition, a posture of a subject P, a body position of the subject P, an X-ray tube angle, and an X-ray detector position. Moreover, the examination protocols are a data file defining, in accordance with a region of a diagnostic target to be examined or an examination purpose, a series of conditions such as setting conditions for an X-ray diagnostic apparatus or a medical image processing apparatus, X-ray conditions, and conditions for imaging technique.

Relative geometric positional relations between a subject P, the X-ray detector 13, and the X-ray tube 31 will also be referred to as first geometric information, the relative geometric positional relations being prescribed by at least a body position of the subject P, an X-ray tube angle, and an X-ray detector position. Specifically, the first geometric information is a geometric positional relation between the subject and the imaging apparatus associated with the examination protocol. The first geometric information includes a body position of a subject P, an angle of the X-ray tube 31, and a position of the X-ray detector 13, those being associated with the examination protocol. The geometric information will be referred to as imaging geometry. The geometric information, which includes the first geometric information and later-described second geometric information, includes an angle of an X ray illuminating the subject P, positions of the X-ray tube 31 and the X-ray detector 13 relative to the subject P, an angle of the X-ray detector 13 relative to the subject P, a range that the X ray illuminates the subject P, a source to image distance (SID), i.e., a distance from the X-ray tube 31 to the subject P, etc. The following description is on this first geometric information.

The first geometric information is parameters associated with an examination protocol. The first geometric information differs depending on the imaged region and the imaging condition. For example, as illustrated in FIG. 3, in a case where the imaged region is a shoulder joint and the imaging condition is "posterior-anterior (PA)", in the prescribed positioning, the body position of the subject P is defined to "Place the shoulder of the subject P to be examined over the X-ray detector 13 and tilt the chest of the subject P relatively to the X-ray detector 13 by 70 degrees". The X-ray tube angle of the X-ray tube 31 is defined to be 20 degrees to a head-to-feet direction of the subject P. The position of the X-ray detector 13 is defined to be on the chest side of the subject P.

The examination protocols herein include an examination protocol, in which the imaged region is a shoulder joint and the imaging condition is "anterior-posterior (AP)". This is an examination protocol created, for example, to enable imaging of the shoulder joint of a subject P in the lying posture when the subject P is unable to be in the standing posture (a normal posture) (for example, for pain due to a bone fracture or for a physical disability). In this case, the body position of the subject P is "Place the shoulder of the subject P over the X-ray detector 13, the shoulder being a shoulder to be not examined, and raise the back of the subject P from the X-ray detector 13 by 70 degrees". In addition, the X-ray tube angle of the X-ray tube 31 is 20 degrees to a feet-to-head direction of the subject P. The position of the X-ray detector 13 is on the back side of the subject P.

Anticipating subjects P who are unable to be imaged in the normal posture, for example, the general X-ray imaging apparatus 1 has plural examination protocols stored in the memory 23 and thereby enables imaging in postures that are tailored to states of the subjects P and do not place physical burdens on the subjects P.

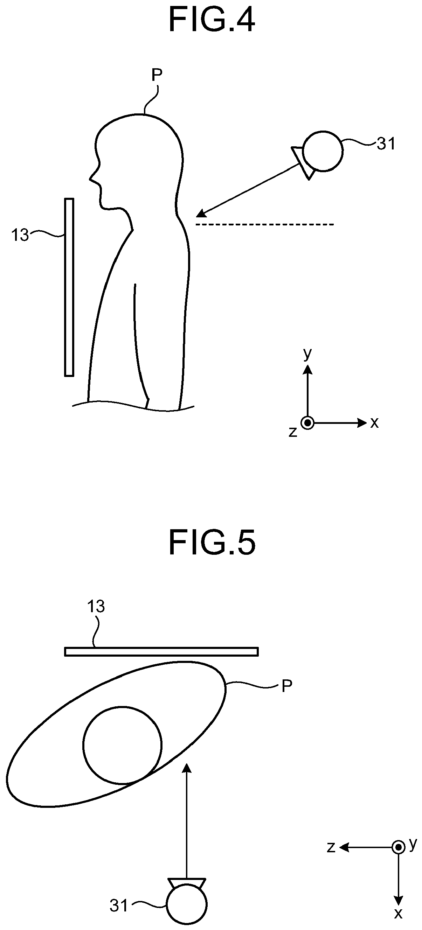

FIG. 4 and FIG. 5 illustrate an example of a positional relation between the X-ray diagnostic apparatus and a subject P in a case where the imaged region is a shoulder joint and the imaging condition is "PA". FIG. 4 illustrates the subject P as viewed from a positive direction along the Z-axis and positional relations between the X-ray detector 13, the X-ray tube 31, and the subject P. FIG. 5 illustrates the subject P as viewed from a positive direction along a Y-axis and the positional relations between the X-ray detector 13, the X-ray tube 31, and the subject P. The parameters of the examination protocols are not limited to this example.

Reference will now be made to FIG. 1 and FIG. 2 again. The second obtainment function 246 obtains a camera image in which a subject P has been captured. Specifically, the second obtainment function 246 obtains a camera image in which a subject P has been captured by the camera 34, the subject P being positioned on the stand 11 in the standing posture or placed on the couchtop 41 in the lying posture, the camera image being stored in the memory 23.

The identification function 247 identifies the second geometric information. Specifically, the identification function 247 analyzes anatomical information (for example, bones, the skeleton, joints, and the respiratory phase) on a subject P from a camera image obtained by the second obtainment function 246 in which the subject P has been captured. On the basis of the anatomical state analyzed, the identification function 247 identifies the second geometric information including a geometric positional relation between the subject P and the imaging apparatus 10 detected based on the camera image in which the subject P has been captured. The second geometric information includes a body position of the subject P, an angle of the X-ray tube 31, and a position of the X-ray detector 13, those being detected based on the camera image in which the subject P has been captured. The anatomical information is identified by a publicly known method from the camera image in which the subject P has been captured. The following description is on identification of the second geometric information from the camera image obtained by the second obtainment function 246 in which the subject P has been captured.

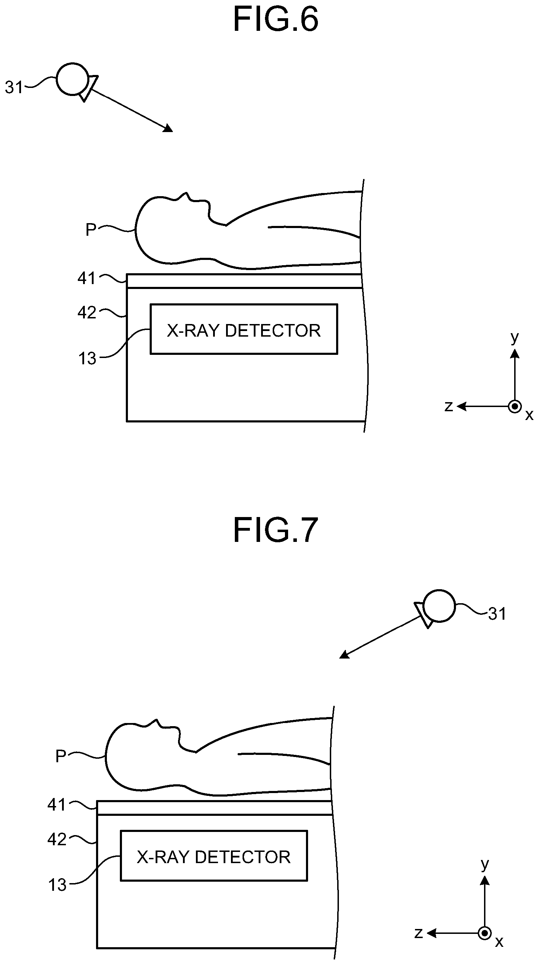

FIG. 6 illustrates the camera image obtained by the second obtainment function 246 in which the subject P has been captured. FIG. 6 illustrates an example of a positional relation between the X-ray diagnostic apparatus and the subject P. FIG. 6 illustrates the subject P as viewed from a positive direction along an X-axis and positional relations between the X-ray detector 13, the X-ray tube 31, and the subject P. The posture of the subject P illustrated in FIG. 6 corresponds to a state where the subject P has been placed on the couchtop 41 in the lying posture. The body position of the subject P corresponds to a state where the back of the subject P is placed over the X-ray detector 13. The X-ray tube angle of the X-ray tube 31 is 20 degrees to the head-to-feet direction of the subject P. The position of the X-ray detector 13 is on the back side of the subject P.

Reference will now be made to FIG. 1 and FIG. 2 again. On the basis of the first geometric information including a geometric positional relation between the subject P and the imaging apparatus 10 associated with the examination protocol obtained by the first obtainment function 245 and the second geometric information including a geometric positional relation between the subject P and the imaging apparatus 10 detected based on the camera image obtained by the second obtainment function 24 in which the subject P has been captured, the determination function 248 determines whether at least one examination item corresponding to the examination protocol is executable.

Specifically, the determination function 248 compares the first geometric information included in the examination protocol obtained by the first obtainment function 245 and the second geometric information identified by the identification function 247 from the camera image obtained by the second obtainment function 24 in which the subject P has been captured with each other and determines whether or not the first geometric information and the second geometric information agree with each other. In a case where the first geometric information and the second geometric information agree with each other, the determination function 248 determines that the at least one examination item corresponding to the examination protocol is executable. On the contrary, in a case where the first geometric information and the second geometric information do not agree with each other, the determination function 248 determines that the at least one examination item corresponding to the examination protocol is not executable.

What is determined by the determination function 248 will now be described specifically. For example, it is now assumed that the imaged region included in an examination protocol obtained by the first obtainment function 245 is a shoulder joint. It is also assumed that the camera image in which the subject P has been captured as illustrated in FIG. 6 is the camera image obtained by the second obtainment function 246 in which the subject P has been captured.

Because the imaged region obtained by the first obtainment function 245 is a shoulder joint, the determination function 248 is able to identify that the posture of the subject P is the standing posture or the lying posture (see FIG. 3). Furthermore, because the posture of the subject P identified by the identification function 247 is the lying posture, the determination function 248 is able to identify that the examination protocol in FIG. 6 has a shoulder joint for the imaged region and "AP" for the imaging condition.

The determination function 248 then compares the first geometric information (see FIG. 3) obtained by the first obtainment function 245 and having the imaged region associated with a shoulder joint and the imaging condition associated with "AP", and the second geometric information identified by the identification function 247, with each other. When the determination function 248 compares the first geometric information and the second geometric information represented by the image of the subject P illustrated in FIG. 6, their body positions of the subject P and their X-ray tube angles of the X-ray tube 31 do not agree with each other.

Specifically, the body positions of the subject P do not agree with each other in that the first geometric information corresponds to a state where "the shoulder to be not examined is placed over the X-ray detector 13 and the back is raised from the X-ray detector 13 by 70 degrees" and the second geometric information corresponds to a state where "the back is placed over the X-ray detector 13".

Furthermore, the X-ray tube angles of the X-ray tube 31 do not agree with each other in that the first geometric information corresponds to a state where the X-ray tube 31 "is 20 degrees to the feet-to-head direction of the subject P" and the second geometric information corresponds to a state where the X-ray tube 31 "is 20 degrees to the head-to-feet direction of the subject P". Therefore, the determination function 248 determines that the examination is not possible because the first geometric information and the second geometric information do not agree with each other.

The output function 249 outputs a result of determination. Specifically, the output function 249 outputs a result of determination performed by the determination function 248, to the display control function 241. In a case where the image of the subject P illustrated in FIG. 6 is an image of a subject P, a result of determination is, for example, "The examination is not possible. The body positions of the subject do not agree with each other and the X-ray tube angles do not agree with each other".

In addition to what has been described above, user assisting information for compliance to the first geometric information included in the examination protocol may be output as the result of the determination. In the case where the image of the subject P illustrated in FIG. 6 is a camera image of a subject P, for example, the user assisting information is, for example, "Set the body position of the subject by placing the shoulder to be not examined over the X-ray detector 13 and raising the back from the X-ray detector 13 by 70 degrees. Set the X-ray tube angle to 20 degrees to the feet-to-head direction of the subject P". The display control function 241 performs control such that the user assisting information is displayed on the display 22. The output function 249 may output the user assisting information with voice or the like to notify the user.

The calculation function 250 calculates a movement position for movement to a position corresponding to an examination protocol. Specifically, in a case the first geometric information associated with an examination protocol obtained by the first obtainment function 245 and the second geometric information detected by the identification function 247 based on a camera image obtained by the second obtainment function 246 in which a subject P has been captured are compared with each other by the determination function 248 and the first geometric information and the second geometric information do not agree with each other, the calculation function 250 calculates a movement position for the X-ray tube 31 or the X-ray detector 13 so that the imaging apparatus 10 is moved to a position corresponding to the first geometric information through movement of at least one of the X-ray tube 31 or the X-ray detector 13. The display control function 241 then performs control such that movement information about movement of the imaging apparatus 10 to the movement position on the display 22. The movement information is information indicating movement of the imaging apparatus 10 to the position corresponding to the first geometric information.

The movement control function 251 performs movement control for movement to a position corresponding to an examination protocol. Specifically, the movement control function 251 performs movement control so that the X-ray tube 31 is moved to a movement position calculated by the calculation function 250. The movement control function 251 may be included in the imaging control function 243.

The movement control performed by the movement control function 251 will now be described specifically. For example, it is now assumed that the imaged region included in an examination protocol obtained by the first obtainment function 245 is a shoulder joint. It is also assumed that the camera image in which the subject P has been captured as illustrated in FIG. 6 is a camera image obtained by the second obtainment function 246 in which a subject P has been captured.

For the image of the subject P illustrated in FIG. 6, the determination function 248 determines that the examination is not possible because the first geometric information and the second geometric information do not agree with each other. The calculation function 250 calculates a movement position so that the X-ray tube 31 is moved to a position corresponding to the first geometric information included in the examination protocol. Furthermore, the movement control function 251 performs movement control so that the X-ray tube 31 is moved to the movement position calculated by the calculation function 250.

Specifically, the movement control function 251 performs movement control so that the X-ray tube 31 is moved from the position (X-ray tube angle) of the X-ray tube 31, "20 degrees to the feet-to-head direction of the subject P", represented by the second geometric information, to the position (X-ray tube angle), "20 degrees to the head-to-feet direction of the subject P", corresponding to the first geometric information. For example, FIG. 7 illustrates an exemplary case in which the X-ray tube 31 is moved to the position of "20 degrees to the head-to-feet direction of the subject P".

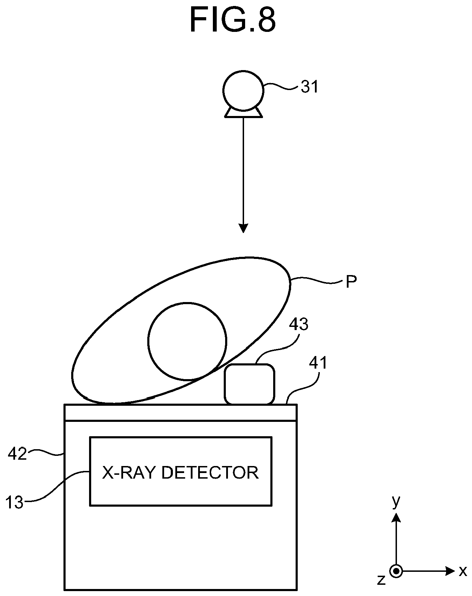

FIG. 7 and FIG. 8 each illustrate an example of a positional relation between the X-ray diagnostic apparatus and a subject P in a case where the movement control function 251 has performed movement control. FIG. 7 illustrates the subject P as viewed from the positive direction along the Z-axis and positional relations between the X-ray detector 13, the X-ray tube 31, and the subject P. FIG. 8 illustrates the subject P as viewed from the positive direction along the Y-axis and the positional relations between the X-ray detector 13, the X-ray tube 31, and the subject P. A support member 43 illustrated in FIG. 8 is a member for raising the back of the subject P. The support member 43 supports the subject P. The support member 43 is adjusted by a radiographer, for example. Specifically, the radiographer looks at the user assisting information displayed on the display screen 22 by the display control function 241. The radiographer then raises the back of the subject P by disposing the support member 43 between the back of the subject P and the couchtop 41.

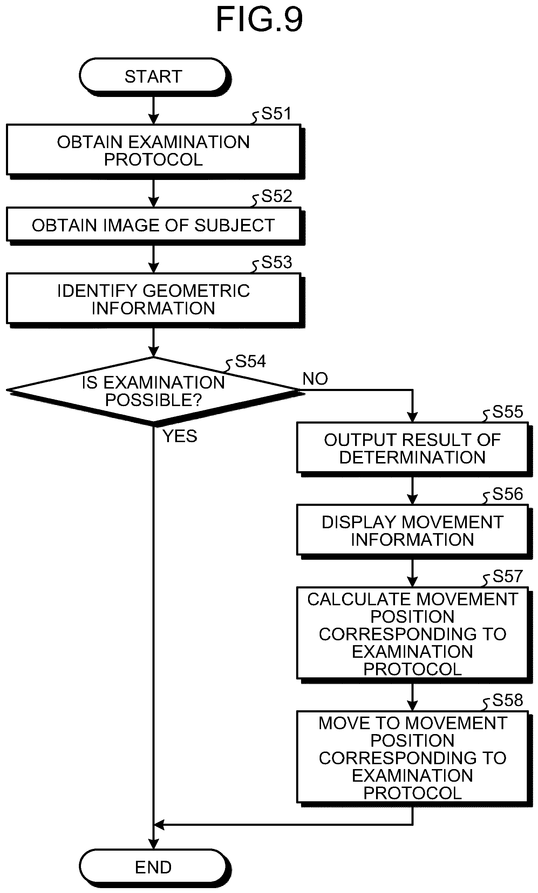

A process executed by the general X-ray imaging apparatus 1 will be described next. FIG. 9 is a flowchart illustrating an example of a process executed by the X-ray diagnostic apparatus according to the embodiment.

Firstly, the first obtainment function 245 obtains an examination protocol stored in the memory 23 (Step S51). Subsequently, the second obtainment function 246 obtains an image of a subject P positioned on the stand 11 in the standing posture or an image of a subject P placed on the couchtop 41 in the lying posture, the image having been captured by the camera 34 and stored in the memory 23 (Step S52) .

Subsequently, the identification function 247 identifies second geometric information from the image of the subject P obtained by the second obtainment function 246 (Step S53). Subsequently, the determination function 248 determines whether or not the examination is possible (Step S54). The process is ended in response to the determination function 248 determining that the examination is possible (Step S54: Yes). On the contrary, in response to the determination function 248 determining that the examination is not possible (Step S54: No), the process is advanced to Step S55.

At Step S55, the output function 249 outputs a result of the determination made by the determination function 248, to the display control function 241 (Step S55). Subsequently, the display control function 241 performs control such that movement information for the imaging apparatus 10 is displayed on the display 22 (Step S56). Specifically, the display control function 241 performs control such that movement information for the X-ray tube 31 is displayed on the display 22. Subsequently, the calculation function 250 calculates a movement position so that the X-ray tube 31 is moved to a position corresponding to the first geometric information included in the examination protocol (Step S57).

Subsequently, the movement control function 251 performs movement control so that the X-ray tube 31 is moved to the movement position calculated by the calculation function 250 (Step S58). In response to Step S58 being ended, the process is ended. After the process is ended, X-ray imaging of the subject P is performed by the imaging control function 243.

As described above, the general X-ray imaging apparatus 1 according to the embodiment obtains: an examination protocol including an imaging condition corresponding to contents of an examination for a subject P; and a camera image in which the subject P has been captured. The general X-ray imaging apparatus 1 then determines whether at least one examination item corresponding to the examination protocol is executable based on first geometric information and second geometric information, the first geometric information including a geometric positional relation between the subject P and the imaging apparatus 10 associated with the examination protocol, and the second geometric information including a geometric positional relation between the subject P and the imaging apparatus 10 detected based on the camera image in which the subject P has been captured.

At least the one embodiment described above enables determination whether the at least one examination item corresponding to the examination protocol obtained is executable, according to a state of the subject P, so that the general X-ray imaging apparatus 1 is able to perform imaging of the subject P in a posture that does not place a physical burden on the subject P even in a case where it is difficult for the subject P to take a posture corresponding to the examination protocol due to pain from a bone fracture or a physical disability, for example.

The subject P is thereby able to take the examination without any physical burden placed on the subject P. Furthermore, an operator who operates the general X-ray imaging apparatus 1 is able to perform the examination even in a case where the subject P is a subject who is unable to take a normal posture. Furthermore, because the apparatus is moved to a position corresponding to the examination protocol even in a case where a normal posture is not taken, the time taken by the operator to perform the examination is shortened. Therefore, the general X-ray imaging apparatus 1 is able to perform imaging of an imaged region of a subject P without placing a physical burden on the subject P, by moving the apparatus according to a state of the subject P.

Moreover, the X-ray imaging apparatus 1 according to the present embodiment performs control such that movement information about movement of the imaging apparatus 10 to a movement position is displayed on a display unit. This allows, for example, a user to dispose a support member for supporting the subject for execution of at least one examination item corresponding to the examination protocol. Furthermore, because the user recognizes movement of the imaging apparatus 10 before the imaging apparatus 10 starts moving, it is possible to prevent the subject P or any peripheral device from hitting against the imaging apparatus 10, which produces safety in the examination.

The embodiment described above may be implemented by modification through change of some of components or functions included in each apparatus, as appropriate. Some modified examples according to the embodiment described above will thus be described hereinafter as other embodiments. Components that are different from those of the embodiment described above will be described mainly hereinafter and detailed description of components that are the same as those described already will be omitted. The modified examples described hereinafter may be implemented individually or may be implemented in combination, as appropriate.

### First Modified Example

According to the above description of the embodiment, the X-ray tube 31 of the general X-ray imaging apparatus 1 is the target subjected to movement control. However, the target subjected to movement control may include, in addition to the X-ray tube 31, the X-ray detector 13.

In this first modified example, the calculation function 250 calculates a movement position for the X-ray tube 31 or the X-ray detector 13 so that the imaging apparatus 10 is moved to a position corresponding to the first geometric information through movement of at least one of the X-ray tube 31 or the X-ray detector. Furthermore, the movement control function 251 performs movement control so that at least one of the X-ray tube 31 or the X-ray detector 13 is moved to the movement position calculated by the calculation function 250.

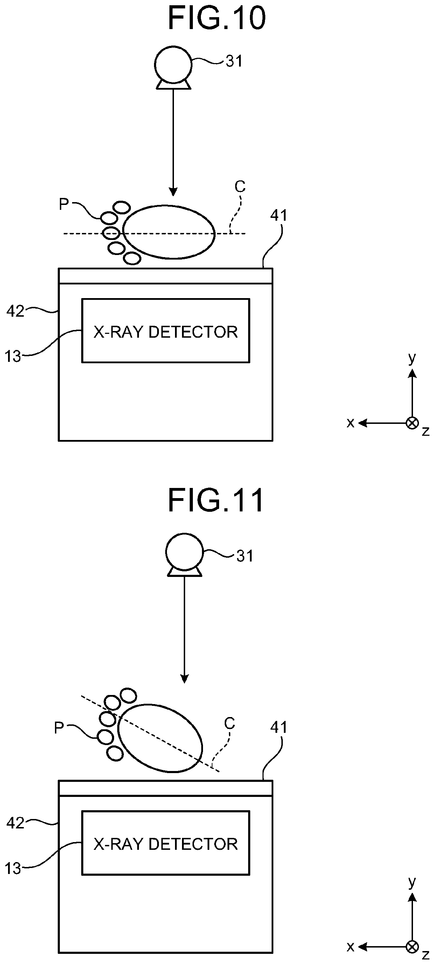

The movement control performed by the movement control function 251 will now be described by use of FIG. 10, FIG. 11, and FIG. 12. FIG. 10, FIG. 11, and FIG. 12 are each a diagram illustrating an example of a positional relation between an X-ray diagnostic apparatus and a subject P, according to the modified example. FIG. 10, FIG. 11, and FIG. 12 each illustrate the subject P as viewed from a negative direction along the Z-axis and positional relations between the X-ray detector 13, the X-ray tube 31, and the subject P. A center line C illustrated in each of FIG. 10, FIG. 11, and FIG. 12 indicates a center position of a foot of the subject P as viewed from the negative direction along the Z-axis.

For FIG. 10, the imaged region written in the table illustrated in FIG. 3, the table illustrating an example of examination protocols, is an ankle joint and the imaging condition written therein is "AP". As illustrated in FIG. 3, in the case where the image region is an ankle joint and the imaging condition is "AP", the posture of the subject P is the lying posture. The body position of the subject P corresponds to a state where the foot of the subject P to be examined is placed over the X-ray detector 13 with the center line of the foot being parallel to the X-ray detector 13. When 0 degree herein indicates a state where the center line of the foot passing through the heel and the toe is perpendicular to the X-ray detector 13, the posture of the subject P is positioned in a state where the center line of the foot makes 90 degrees. Furthermore, the X-ray tube angle of the X-ray tube 31 is perpendicular to the X-ray detector 13. The X-ray detector position of the X-ray detector 13 is on the back side of the subject P.

FIG. 11 illustrates, for example, a case where the subject P is unable to maintain a normal posture. Specifically, the body position of the subject P corresponds to a state where the foot of the subject P to be examined is placed over the X-ray detector 13 while the center line C of the foot is unable to be parallel to the X-ray detector 13. In other words, the posture of the subject P fails to be positioned such that the center line C of the foot makes 90 degrees, and it is in a state where the center line C of the foot makes an angle larger than 0 degree and smaller than 90 degrees. Furthermore, the X-ray tube angle of the X-ray tube 31 is perpendicular to the X-ray detector 13. The X-ray detector position of the X-ray detector 13 is on the back side of the subject P.

The examination is not possible in the body position of the subject P illustrated in FIG. 11 and the calculation function 250 thus calculates a movement position so that the movement control function 251 moves, in a state where the body position of the subject P has been maintained, the X-ray tube 31 and the X-ray detector 13 to a position corresponding to the first geometric information included in the examination protocol. The movement control function 251 then performs movement control so that the X-ray tube 31 and the X-ray detector 13 are moved to the movement position calculated by the calculation function 250.

Specifically, the movement control function 251 performs movement control so that the X-ray detector 13 becomes parallel to the center line C of the subject P. Furthermore, the movement control function 251 performs movement control so that the X-ray tube 31 becomes perpendicular to the X-ray detector 13.

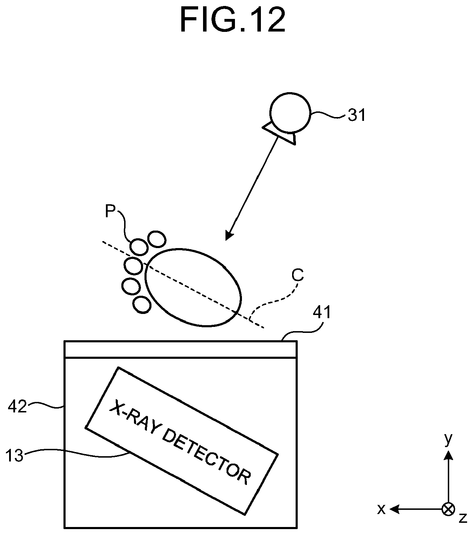

FIG. 12 illustrates a state after the movement control function 251 has performed the movement control of the X-ray tube 31 and the X-ray detector 13. The subject P illustrated in FIG. 12 is in a state similar to the state according to the first geometric information in the case illustrated in FIG. 3 where the imaged region is an ankle joint and the imaging condition is "AP" and thus the examination is possible and the general X-ray imaging apparatus 1 is able to implement imaging of the subject P. Note that, as illustrated in FIG. 12, the couchtop 41 may not move while only the X-ray detector 13 may move. In other words, the movement control function 251 may perform movement control over the X-ray tube 31 such that the X-ray tube 31 becomes perpendicular to the X-ray detector 13.

As described above, on the basis of at least one executable examination item, the general X-ray imaging apparatus 1 according to this modified example performs movement control so that at least one of the X-ray tube 31 or the X-ray detector 13 is moved to a position corresponding to first geometric information included in an examination protocol.

In the modified example described above, movement control is performed so that at least one of the X-ray tube 31 or the X-ray detector 13 is moved to a position corresponding to first geometric information associated with an examination protocol, according to a camera image in which a subject P has been captured, so that the general X-ray imaging apparatus 1 is able to perform imaging of the subject P in a posture that places no burden on the body of the subject P even in a case where it is difficult for the subject P to take a posture corresponding to the examination protocol due to pain from a bone fracture or a physical disability, for example.

The subject P is thereby able to take the examination without any physical burden placed on the subject P. Therefore, the general X-ray imaging apparatus 1 is able to perform imaging of an imaged region of the subject P without placing any physical burden on the subject P, by moving the apparatus according to a camera image in which the subject P has been captured.

At least the embodiment and modified example described above enable examinations without placing burdens on subjects P.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope spirit of the invention as defined by the appended claims.

## Claims

1. An X-ray diagnostic apparatus comprising:
an imaging apparatus including
an X-ray tube configured to generate an X ray; and
an X-ray detector configured to detect an X ray that has been emitted from the X-ray tube and passed through a subject;
a first obtainment unit (245) configured to obtain an examination protocol including an imaging condition corresponding to contents of an examination for the subject;
a second obtainment unit (246) configured to obtain a camera image in which the subject has been captured; and
a determination unit (248) configured to determine whether at least one examination item corresponding to the examination protocol is executable based on first geometric information and second geometric information, the first geometric information including a geometric positional relation between the subject and the imaging apparatus associated with the examination protocol, and the second geometric information including a geometric positional relation between the subject and the imaging apparatus detected based on the camera image.

2. The X-ray diagnostic apparatus according to claim 1, further comprising an execution unit (243) configured to execute the at least one executable examination item, wherein when the at least one examination item corresponding to the examination protocol is determined to be executable, the execution unit (243) is configured to execute the executable item.

3. The X-ray diagnostic apparatus according to claim 1, wherein the determination unit (248) is configured to determine whether the first geometric information and the second geometric information agree with each other, and when the first geometric information and the second geometric information do not agree with each other, determine the at least one examination item corresponding to the examination protocol to be not executable, and the X-ray diagnostic apparatus further comprising
a movement control unit (251) configured to control at least one of the X-ray tube or the X-ray detector such that the imaging apparatus is moved to a position corresponding to the first geometric information.

4. The X-ray diagnostic apparatus according to claim 1, wherein
the first geometric information includes a body position of the subject, an angle of the X-ray tube, and a position of the X-ray detector, those being associated with the examination protocol, and
the second geometric information includes a body position of the subject, an angle of the X-ray tube, and a position of the X-ray detector, those being detected based on the camera image.

5. The X-ray diagnostic apparatus according to claim 3, further comprising a calculation unit (250) configured to calculate a movement position for the X-ray tube or the X-ray detector such that the imaging apparatus is moved to the position corresponding to the first geometric information by moving at least one of the X-ray tube or the X-ray detector.

6. The X-ray diagnostic apparatus according to claim 3, further comprising a display control unit (241) configured to perform control such that movement information about movement of the imaging apparatus to a movement position is displayed on a display unit.

7. The X-ray diagnostic apparatus according to claim 1, further comprising an optical camera capable of collecting the camera image in a same direction as the X ray illuminates in an X-ray tube retaining unit that retains the X-ray tube.

8. An X-ray diagnostic method performed by an X-ray diagnostic apparatus that includes an imaging apparatus including an X-ray tube configured to generate an X ray and an X-ray detector configured to detect an X ray that has been emitted from the X-ray tube and passed through a subject, the X-ray diagnostic method comprising:
a first obtainment step of obtaining an examination protocol including an imaging condition corresponding to contents of an examination for the subject;
a second obtainment step of obtaining a captured image in which the subject has been captured; and
a determination step of determining whether at least one examination item corresponding to the examination protocol is executable based on first geometric information and second geometric information, the first geometric information including a geometric positional relation between the subject and the imaging apparatus associated with the examination protocol, and the second geometric information including a geometric positional relation between the subject and the imaging apparatus detected based on the captured image.
